(19) **Europäisches Patentamt European Patent Office Office européen des brevets**

(11) **EP 4 752 212 A2**

(12) **EUROPEAN PATENT APPLICATION**
published in accordance with Art. 153(4) EPC

(43) Date of publication:
**03.06.2026 Bulletin 2026/23**

(21) Application number: 24773495.7

(22) Date of filing: **12.07.2024**

(51) International Patent Classification (IPC):
*C12M 3/00* (2006.01)   *C12M 1/42* (2006.01)
*C12M 1/34* (2006.01)   *C12M 1/36* (2006.01)
*G01N 33/483* (2006.01)

(52) Cooperative Patent Classification (CPC):
**C12M 35/04; C12M 21/08; C12M 41/46; C12M 41/48**

(86) International application number:
**PCT/ES2024/070443**

(87) International publication number:
**WO 2025/022027 (30.01.2025 Gazette 2025/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:  **27.07.2023  ES 202330648**

(71) Applicants:
• **Universidad De Granada**
  **18071 Granada (ES)**
• **Bioiberica, S.A.U.**
  **08389 Palafolls Barcelona (ES)**

(72) Inventors:
• **MARTÍNEZ MORENO, Daniel**
  **18071 Granada (ES)**
• **MARCHAL CORRALES, Juan Antonio**
  **18071 Granada (ES)**
• **RUS CARLBORG, Guillermo**
  **18071 Granada (ES)**
• **CALLEJAS ZAFRA, Antonio Manuel**
  **18071 Granada (ES)**
• **JIMÉNEZ GONZÁLEZ, Gema**
  **18071 Granada (ES)**
• **GÁLVEZ MARTÍN, Patricia**
  **08389 Palafolls Barcelona (ES)**

(74) Representative: **Pons IP**
  **Glorieta Rubén Darío 4**
  **28010 Madrid (ES)**

(54) **METHOD AND BIOREACTOR FOR DETERMINING IN REAL TIME THE GROWTH AND MATURATION OF A BIOLOGICAL TISSUE**

(57)    The bioreactor comprises: a cell culture chamber configured to house a structure for accommodating a cell source and intended to promote cell culture; a transmitting transducer configured to emit an ultrasonic wave in the direction of the structure; a receiving transducer facing the transmitting transducer and configured to receive the ultrasonic wave once it has passed through the structure; and a fluid circuit intended to pass through the structure in order to generate a shear stress on the surface thereof to facilitate the generation and differentiation of cells, wherein the fluid circuit comprises a peristaltic pump.

EP 4 752 212 A2

**Description**

**OBJECT OF THE INVENTION**

The present invention belongs to the field of cell culture, more specifically to the field of bioreactors with structures adapted for cell culture.

The invention relates to a method for determining in real time the growth and maturation of a biological tissue. Furthermore, the present invention relates to a method for optimising the production of a biological tissue based on the measurements of the method for determining in real time the growth and maturation of a biological tissue.

The invention also relates to a bioreactor for generating a biological tissue by cell culture, comprising elements configured to carry out the previous methods, and a system for generating the cell culture that comprises the bioreactor. The bioreactor of the invention makes it possible to monitor and optimise the generation and differentiation of cells for generating biological tissues.

**BACKGROUND OF THE INVENTION**

The aim of regenerative medicine (RM) is to restore or establish normal function through the complete or partial regeneration of human cells, organs or tissues. Living cells, biomaterials and bioactive signals are the three pillars of tissue engineering (TE).

One of the several diseases that can benefit from advances in TE is osteoarthritis (OA), an incurable, complex disorder. OA is an important socioeconomic disease that mainly affects the articular cartilage (AC), which is a tissue with a low self-healing capacity and, therefore, an ideal target for RM and TE.

The pathogenic process that leads to OA is characterised by persistent low-grade AC impairment, which is the main cause of continued joint degeneration. Due to loss of AC, OA causes pain and loss of joint function. As such, OA should not be considered a disease, but rather as the common endpoint of a series of secondary pathways associated with age, possible traumas, obesity and the resulting changes in joint biomechanics.

A mechanical disorder that causes low-grade lesions to the AC is the main driving force in the development of OA. As a result, three different stages can be identified from a biomechanical perspective: i) the proteolytic breakdown of the extracellular matrix (ECM) of the AC, ii) fibrillation and erosion of the AC surface, and iii) the onset of synovial inflammation.

A common mechanical stimulation for AC are mechanical loads, which are described as direct interactions between two surfaces, in the form of stresses that vary from 0.5 to 8 MPa. Likewise, friction loads exerted by the interstitial fluid increase the pressurisation of the cartilaginous fluid, inducing hydrostatic pressures. This fact contributes to the increase in the stiffness of the AC under dynamic stresses. Furthermore, the solid ECM sustains the remaining proportion (66%) of the compression load.

Since the AC is an avascular tissue without a lymphatic system or nerve endings, patients with OA have a limited regeneration rate, where TE is a precise tool for reversing this process with techniques such as biomanufacturing. There is currently no known treatment for OA that can stop or slow its progression.

Currently, AC injuries are mainly treated with surgical treatments. However, these treatments lack long-term effectiveness. Another example is mosaicplasty, a treatment for focal chondral lesions which results are relatively acceptable during the first 2 years, but in many cases it later fails.

Numerous TE-based products and treatments have attempted to simulate AC for some decades, including autologous chondrocyte implantation (ACI) and matrix autologous chondrocyte implantation (MACI).

The biomanufacturing of structures that simulate tissues and/or organs for their use in TE is carried out using synthetic biomaterials such as polylactic acid (PLA), polycaprolactone (PCL) and polylactic-co-glycolic acid (PLGA). These materials are not able to easily imitate the mechanical properties of real tissue. Therefore, the use of new biomaterials that withstand stresses are quite promising in TE for OA. Nonetheless, it is important to ensure adequate cell-biomaterial interaction by reducing the hydrophobic behaviour of this type of synthetic polymers. A good way to address this problem is through surface modifications, for example, with Arg-Gly-Asp (RGD) peptides, with 1-pyrenebutyric acid (PBA) or with several ECM elements such as fibronectin or collagen.

Previous research has been conducted to determine how biomechanical stimulation induces chondrogenesis of mesenchymal stem cells (MSC) by phosphorylation of Sox9 through protein kinase A (PKA), cAMP, Ser133 and CREB. Therefore, any pathway that involves the transmission of signals from mechanical stimulation to electrochemical activity is known as mechanotransduction. The mechanosensory transient receptor potential vanilloid 4 (TRPV4), Piezo1 and Piezo2 are found in chondrocytes and osteoblasts. Both bone tissue and cartilage tissue exhibit acute mechanosensitivity to maintain homeostasis. For example, osteoporosis is related to the inhibition of Piezo1, and TRPV4 regulates the anabolic response of chondrocytes to osmotic or mechanical stress. Although the exact mechanism of action is still unknown, some evidence points to primary cilia as a mechanosensory potential for shear forces.

As explained, to date, ineffective interventions have been developed for the treatment of AC lesions. Therefore, novel bioengineered medical devices that induce *ex vivo* maturation of biomimetic structures of biological tissues, such as cartilage, in three dimensions are imperative.

## DESCRIPTION OF THE INVENTION

In one aspect, the present invention relates to a method for determining in real time the growth and maturation of a biological tissue, hereinafter, "determination method of the invention", and comprising:

- introducing a flow of culture medium into a bioreactor, wherein the bioreactor comprises:

    ○ a culture chamber (1) that houses a structure (5), which accommodates a cell source,
    ○ a transmitting transducer (2) and a receiving transducer (3) located opposite the transmitting transducer (2), and
    ○ a fluid circuit (4) connected to the culture chamber (1), located between the transmitting transducer (2) and the receiving transducer (3), and through which the flow of culture medium destined to pass through the structure (5) is introduced;

- generating an ultrasonic wave by means of the transmitting transducer (2) that passes through the culture chamber (1) and the structure (5) with the cell source housed in said culture chamber (1);
- receiving the ultrasonic wave in the receiving transducer (3) once it has passed through the structure (5), generating a reception signal (3);
- generating an ultrasound propagation simulation model, calculating a simulated reception signal and setting density of the structure (5) and speed of sound in the structure (5) as dependent variables;
- defining a distance metric between the simulated reception signal and the reception signal (3) of the ultrasonic wave obtained in the receiving transducer (3),
- determining the density in the structure (5) and the speed of sound in the structure (5) that minimises the distance metric, at each moment of time; and
- correlating the density and speed of sound that are obtained with an increase in stiffness and tissue content, thus monitoring tissue growth over time.

In a preferred embodiment of the determination method of the invention, the simulation model is generated using finite element models (FEM), finite differences, a transfer-matrix method (transfer-matrix formalism) or spectral techniques.

When generating the ultrasound propagation simulation model, setting boundary conditions can be specified depending on the geometry, type of tissue and wave. Thus, the boundary conditions can be set routinely by the person skilled in the art.

Furthermore, when calculating the simulated signal, the following variables are preferably simulated: the displacement matrix of the transducers (where the variables are X,Y), the pressure gradient across the propagation fields (acoustic wave, variable in Pa), and the voltage of the receiving transducer is also simulated through the piezoelectric effect.

In another preferred embodiment of the determination method of the invention, the distance metric used is a cross-correlation between the signals, a sum of the components of the vector or a square root of the sum of the squares of the components of the vector.

In the present invention, the distance metric refers to phase or time of flight (ToF).

In another preferred embodiment of the determination method of the invention, the step of determining the density and propagation speed is carried out using a search algorithm that may be a gradient-based method, a genetic algorithm or a neural network trained with a set of pre-calculated receiving ultrasound signals and values of density and speed of sound corresponding to said signals.

In the present invention, the expression "growth and maturation of a biological tissue" refers to the process by which cells from the cell source duplicate, differentiate and/or produce elements of the extracellular matrix. The extracellular matrix is composed of a complex mixture of structural biomolecules that are released by a cell into the extracellular medium. Extracellular matrices are involved in supporting the cells that are part of a tissue. Extracellular matrices comprise fibrous elements which include collagen fibres and elastic fibres. Furthermore, extracellular matrices include glycoproteins, such as basement membrane laminin, microfibrils around elastic fibres, fibres, fibronectins on the cell surface and the like. The particularly differentiated tissue has the same basic structure. For example, in hyaline cartilage, chondroblasts characteristically produce a large number of cartilage matrices that include proteoglycans. In the bones, osteoblasts produce bone matrices that cause calcification.

In the present invention, the variables that correlate with the growth and maturation of biological tissue are the measurements of density and ultrasound speed that are obtained by the determination method of the invention. Increased cell number and increased extracellular matrix production correlate with increased density and increased speed of sound.

In the present invention, the term "bioreactor" refers to a device that comprises the elements necessary to carry out the cell culture, such as a culture chamber (1) that houses a structure (5) which accommodates a cell source, and a fluid circuit (4) that provides a culture medium.

The flow of culture medium introduced into the culture chamber (1) of the bioreactor in the determination method of the invention not only provides the necessary nutrients for the cell source, but also generates mechanical stimulation that induces the growth and maturation of a biological tissue. The flow of culture medium can be introduced through a fluid circuit (4).

In the present invention, the term "biological tissue" refers to the aggregation of one or more types of cells together to perform a particular function in an organism. Biological tissues are part of organs or different physiological systems of a subject, where subject refers to a human or a non-human animal (e.g., mouse, rat, dog, pig or horse). The present invention specifically relates to solid tissues that include, but are not limited to, bone tissue or bone, cartilage in the skeletal system or an organ, muscle, adipose tissue, epithelial, connective or nervous tissue. The different types of tissue are not limited to normal anatomical tissues, but can also include different types of specialised cells under pathological conditions, as tumour and adjacent non-tumour tissue of the same or different type, such as breast adenocarcinoma and adjacent normal (non-malignant) breast tissue. The application of mechanical stimulation is one of the necessary factors in the generation and differentiation of tissue cells such as cartilage tissue or bone tissue, but it also promotes the generation and differentiation of other solid biological tissues such as muscle tissue, myocardium and vascular tissue, among others (Ogunrinade, O., Kameya, G. T., & Truskey, G. A. (2002). Effect of fluid shear stress on the permeability of the arterial endothelium. Annals of biomedical engineering, 30, 430-446; Yeatts, A. B., & Fisher, J. P. (2011). Bone tissue engineering bioreactors: dynamic culture and the influence of shear stress. Bone, 48(2), 171-181; Lee, A. A., Graham, D. A., Dela Cruz, S., Ratcliffe, A., & Karlon, W. J. (2002). Fluid shear stress-induced alignment of cultured vascular smooth muscle cells. J. Biomech. Eng., 124(1), 37-43; Huang, Y., Jia, X., Bai, K., Gong, X., & Fan, Y. (2010). Effect of fluid shear stress on cardiomyogenic differentiation of rat bone marrow mesenchymal stem cells. Archives of medical research, 41(7), 497-505). The determination method of the present invention makes it possible to determine the growth and maturation of biological tissue based on the measured changes in density and speed of sound, given by changes in the number of cells and production of elements of the extracellular matrix. The determination method of the present invention is applicable to different types of tissues taking into account the final density of each type of tissue, wherein said density is given by mass of tissue/volume of tissue (e.g., g of tissue/$cm^3$ of tissue).

In the determination method of the invention, the conditions of temperature, oxygen and carbon dioxide inside the culture chamber (1) of the bioreactor can be routinely defined by the person skilled in the art, depending on the type of biological tissue that is to be generated.

The culture chamber (1) in the bioreactor of the method of the invention houses a structure (5) which accommodates a cell source for generating biological tissue, promoted by the mechanical stresses produced by the flow of culture medium.

In the present invention, the term "cell source" refers to the set of cells used for generating a tissue by applying the conditions and stimuli necessary for it. The cell source is therefore under optimal temperature, pressure and nutrient supply conditions for its culture, said variables being routinely defined by the person skilled in the art.

In the present invention, the term "culture medium" refers to a liquid solution that is used to provide sufficient nutrients (e.g., vitamins, amino acids, essential nutrients, salts and the like) and has the properties (e.g., osmolarity, buffering) to keep cells alive (or living cells in a tissue) and promote their growth and/or differentiation.

In the present invention, the cell source can be of different types and origins, which, through appropriate culture conditions, give rise to a specific type of biological tissue.

Therefore, in a preferred embodiment of the determination method of the invention, the cell source is selected from stem cells and differentiated cells.

The term "stem cells", as used herein, refers to undifferentiated, self-renewing cells that are capable of differentiating into one or more types of specialised cells. Stem cells can be found in both embryonic and adult tissues, being embryonic stem cells or adult stem cells, respectively. Adult stem cells contain mesenchymal stem cells (MSCs), which can be obtained from tissues such as adipose tissue or bone marrow, among others. (MSCs are a source of undifferentiated cells that can become the cell lineage of different tissues, such as the chondrogenic lineage (e.g., chondroblasts) or osteogenic lineage (e.g., osteoblasts). MSCs have a high *in vitro* proliferation rate, preserving their multipotent differentiation capacity, which makes them quite interesting as therapeutic agents. The infrapatellar fat pad is one of the sites from which multipotent cells can be isolated, for generating biological tissues using tissue engineering (TE) techniques and means, such as those of the present invention. Stem cells can also be obtained through cell reprogramming techniques in the laboratory, obtaining the so-called induced pluripotent stem cells, which can also be used as a cell source.

Therefore, in another preferred embodiment of the determination method of the invention, the cell source is made of stem cells selected from the list consisting of: adult stem cells, embryonic stem cells and induced pluripotent stem cells.

Furthermore, in the present invention, the term "differentiated cells" refers to those cells that, after a maturation process, exhibit functional characteristics typical of the tissue of which they are part; moreover, differentiated cells are characterised by having lost their capacity to naturally differentiate into other types of specialised cells. Examples of differentiated cells include, but are not limited to, fibroblasts, chondrocytes, osteocytes, myocytes or neurons.

Therefore, in another preferred embodiment of the determination method of the invention, the differentiated cells are selected from the list consisting of: fibroblasts, chondrocytes, chondroblasts, osteocytes, osteoblasts, myocardiocytes, endothelial cells and myocytes.

In a more preferred embodiment of the determination method of the invention, the biological tissue is cartilage tissue.

In another even more preferred embodiment of the determination method of the invention, the biological tissue is cartilage tissue, and the cell source is selected from mesenchymal stem cells and differentiated cells; wherein the differentiated cells are selected between chondrocytes and fibroblasts.

Both stem cells and differentiated cells can be obtained from the tissues of a subject, wherein the subject can be a human or a non-human animal. Both stem cells and differentiated cells are preferably obtained from the tissues of the subject, who will subsequently receive a graft of the generated biological tissue.

The structure (5) in which the cell source is accommodated to promote the cell culture and the generation of biological tissue can be made up of any biomaterial that allows adequate stiffness and consistency to form fibrillar networks to adapt cell adhesion, cell viability and flow passage through its pores. Some examples of biomaterials that make up a structure (5) for accommodating the cell source include, but are not limited to, synthetic polymers such as 1,4-butanediol thermoplastic polyurethane (bTPUe), or polylactic acid (PLA), polycaprolactone (PLC) or polylactic-co-glycolic acid (PLGA). The provided examples of synthetic polymers are within the scope of protection of the determination method of the invention.

To ensure adequate cell-biomaterial interaction in the structure (5), by reducing the hydrophobic behaviour of synthetic polymer materials such as bTPUe, PLA, PLC or PLGA, the structure (5) that accommodates the cell source within the bioreactor can be previously functionalised, for example, with Arg-Gly-Asp (RGD) peptides, with 1-pyrenebutyric acid (PBA), 1-pyreneacetic acid (PCA), or with several ECM elements such as fibronectin or collagen.

The biomaterial of the structure (5), which accommodates the cell source in the determination method of the invention, forms a network of meshed fibres, which comprises gaps in which the cells of the cell source are located.

Therefore, in a preferred embodiment of the determination method of the invention, the structure (5) is made up of fibres with a diameter between 50 $\mu$m and 400 $\mu$m and is represented in the simulation model as a matrix of circles.

In another preferred embodiment of the determination method of the invention, wherein the transmitting transducer (2) and the receiving transducer of the structure (5) are facing each other, and both are also placed perpendicular to the fluid circuit (4). Therefore, the direction of the ultrasonic wave generated by the transmitting transducer (2) is parallel to the direction of the flow of the culture medium passing through the structure (5).

The material of the walls of the bioreactor culture chamber (1) can be any bioinert material that has a density and impedance significantly different from the structure (5) with the cell source that is housed in the culture chamber (1).

As the growth and maturation of biological tissue occurs, variations occur in the diameter of the fibres of the structure (5). The variation in diameter of the fibres of the structure (5) is due to an increase in the number of cells of the cell source and an increase in the extracellular matrix produced by them. Therefore, as the growth and differentiation of biological tissue occurs, the number of cells and extracellular matrix increases, and therefore the diameter of the fibres of the structure (5) increases. By increasing the diameter of the structure (5), the flow of culture medium progressively decreases, thus possibly affecting the production of biological tissue.

Therefore, the present inventors have also developed a method for optimising the production of biological tissue based on the determinations carried out in the determination method of the invention.

Thus, in another aspect, the invention relates to a method for optimising the production of biological tissue, hereinafter, "optimisation method of the invention", comprising the steps of:

- determining a density of the structure (5) and a speed of sound in the structure (5) using the determination method of the invention; and
- determining a pump flow that optimises the growth and maturation of tissue through the expression:

$$ q = u \cdot \left(1 - \frac{\pi \cdot (K_4 \cdot \rho_{ECM})^2}{4 \cdot A_{total}}\right) $$

where q is the pump flow, u is the flow speed and is constant, $K_4$ is a constant that relates a variation in the diameter of fibres of the structure with a matrix density, $\rho_{ECM}$ is the extracellular matrix density and $A_{total}$ is the total area of the structure (5) in a direction perpendicular to the pump flow.

In the present invention, the term "pump flow" refers to the flow of culture medium pumped by a pump, preferably a peristaltic pump, and introduced into the bioreactor through the fluid circuit (4).

In a preferred embodiment of the optimisation method of the invention, $K_4$, which relates a variation in the diameter of fibres of the structure with a matrix density, is obtained through the expression:

$$K_4 = \left( K_2 + \frac{K_3}{K_1} \right)$$

where $K_4$, is obtained from the expressions:

$$\frac{dD_f(t)}{dt} = K_2 \cdot \rho_{ECM} + K_3 \cdot [cell] - \zeta_{material}$$

$$\rho_{ECM} = K_1[cell]$$

and where $K_1$ is a constant that depends on cell metabolism, the type of matrix synthesised and the age of the cells, [cell] is the concentration of cells attached to the structure, $D_f$ is the diameter of the fibre of the structure and varies depending on: $K_2 \cdot \rho_{ECM}$ which represents the matrix synthesised by cells, where $K_2$ is the diameter increase coefficient as a function of the amount of matrix, $K_3 \cdot [cell]$ which represents the number of attached cells, where $K_3$ is the diameter increase coefficient as a function of the cell mass, and $\zeta_{material}$ which represents the degradation of the polymer that makes up the structure (5) and which is considered negligible.

Some results achieved that allow the simulated signal of the computational model to be correlated with the measured ultrasound signal to determine the growth of the cell source on the structure are:

-    the received voltage decreases and a delay occurs in the received signal;
-    the amplitude of the received signal increases;
-    the time of flight (ToF) signal increases;
-    the component K of the speed of sound equation increases more than density, indicating an increase in the elastic modulus or a decrease in viscosity; and
-    there is an absence of considerable close bounces.

In another aspect, the present invention relates to a bioreactor for generating biological tissue, hereinafter, "bioreactor of the invention", which is intended to accommodate a cell source and comprises:

-    a cell culture chamber (1) configured to house a structure (5) to accommodate the cell source;
-    a transmitting transducer (2) configured to emit an ultrasonic wave in the direction of the structure (5);
-    a receiving transducer (3) arranged opposite the transmitting transducer (2) and configured to receive the ultrasonic wave once it has passed through the structure (5);
-    a fluid circuit (4) intended to pass through the structure (5) in order to generate a shear stress on the surface thereof to facilitate the generation and differentiation of cells; wherein the fluid circuit (4) comprises an inlet (6) and an outlet (7), and a peristaltic pump (8) configured to generate a laminar flow; and
-    a processing module configured to carry out the steps of the determination method of the invention, and depending on the density in the structure (5) and the speed of sound in the structure (5) that are obtained, controlling the flow generated by the peristaltic pump (8) by regulating the generated pump flow, according to the steps of the method according to the optimisation method of the invention.

In another preferred embodiment, the bioreactor of the invention further comprises a signal generator (9) connected to the transmitting transducer (2); preferably, the signal generator (9) is further connected to the processing module to generate the ultrasonic wave.

In another preferred embodiment, the bioreactor of the invention further comprises an amplifier (10) and an oscilloscope (11) that are connected to the receiving transducer (3).

In another preferred embodiment of the bioreactor of the invention, the transmitting transducer (2) and the receiving transducer of the structure (5) are facing each other and placed perpendicular to the fluid circuit (4). Therefore, the direction of the ultrasonic wave generated by the transmitting transducer (2) is parallel to the direction of the flow of the culture medium passing through the structure (5).

In another preferred embodiment of the bioreactor of the invention, the processing module is a Raspberry Pi (12) and an Arduino board (13) connected to the peristaltic pump (8).

In another preferred embodiment, the bioreactor of the invention further comprises a container (14) intended to store the culture medium and connected to the peristaltic pump (8), to the inlet (6) of the fluid circuit (4) and to the outlet (7) of the fluid circuit (4).

In another preferred embodiment of the bioreactor of the invention, the culture chamber (1) is made of a material selected from the list consisting of: polymethyl methacrylate (PMMA), stainless steel, titanium, sapphire crystal, platinum, and glass.

In another aspect, the present invention relates to a system for generating biological tissue, hereinafter, system of the invention, which comprises the bioreactor of the invention and further comprises a structure (5) located in the cell culture chamber (1), on which the cell source is arranged.

In a preferred embodiment of the system of the invention, the structure (5) is made of a polymer selected from the list consisting of: 1,4-butanediol thermoplastic polyurethane (bTPUe), polylactic acid (PLA), polycaprolactone (PLC) and polylactic-co-glycolic acid (PLGA).

In another preferred embodiment of the system of the invention, the structure (5) is made up of fibres with a diameter of between 50 $\mu$m and 400 $\mu$m.

In another preferred embodiment of the system of the invention, the surface of the structure (5) is functionalised with Arg-Gly-Asp (RGD) peptides, with PBA, 1-pyrene acetic acid (PCA) with fibronectin or with collagen.

In another aspect, the present invention relates to a computer program, hereinafter, "program of the invention", for determining in real time the growth and maturation of a biological tissue configured to carry out the steps of the determination method of the invention, and for optimising the production of tissue configured to carry out the steps of the optimisation method of the invention by using the system of the invention.

In another aspect, the present invention relates to a storage unit configured to store the computer program of the invention.


## DESCRIPTION OF THE DRAWINGS

To complement the description that is being made and for the purpose of helping to better understand the features of the invention, according to a preferred practical exemplary embodiment thereof, a set of figures is attached as an integral part of said description wherein the following has been depicted in an illustrative and non-limiting manner:

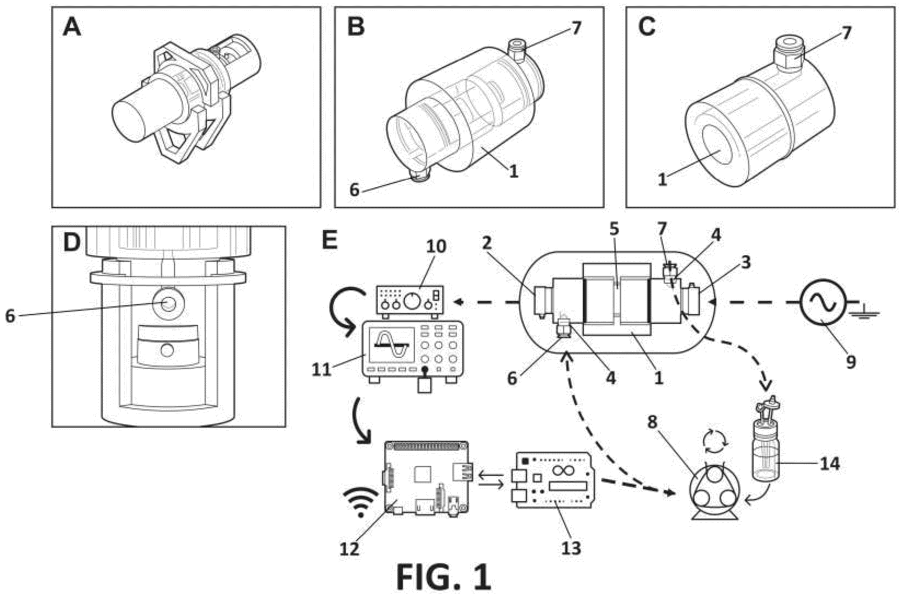

Figure 1. It shows the elements of the bioreactor (BR) according to a preferred embodiment of the invention. A) View of the bioreactor. B) View of the bioreactor culture chamber (1), inlet (6) and outlet (7) of the fluid system. C) View of the culture chamber (1) with the outlet of the flow system (7). D) Visual concept of the transducer with the coupling system. E) Diagram showing the different elements of the bioreactor: culture chamber (1), transmitting transducer (2) arranged opposite the receiving transducer (3), fluid circuit (4) intended to pass through the structure (5) that accommodates the cell source, fluid circuit inlet (6), fluid system outlet (7), peristaltic pump (8), signal generator (9), amplifier (10), oscilloscope (11), Raspberry Pi (12), Arduino board (13), and container (14) intended to store the culture medium and connected to the peristaltic pump (8).

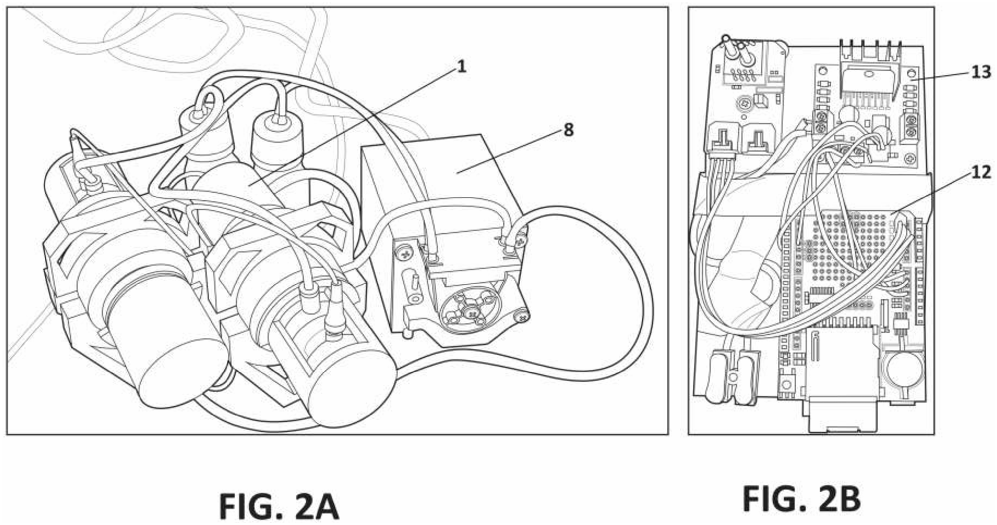

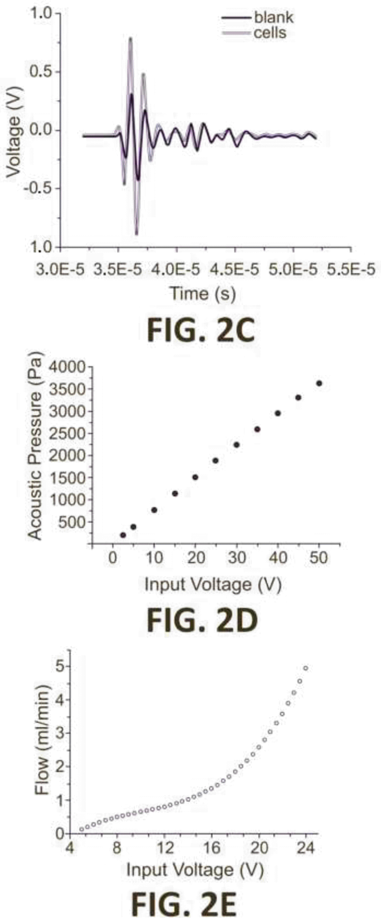

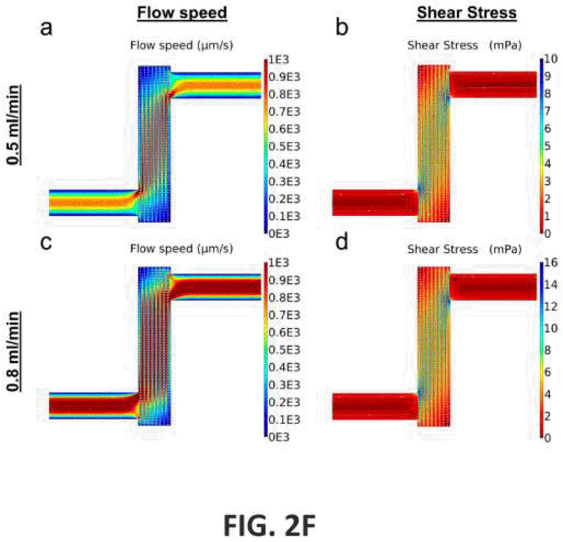

Figure 2. Final layout of the bioreactor (BR) and global parameters. (A) Photograph of the final assembly of the BR (1) inside a laminar hood, peristaltic pump (8). (B) Photograph of the Arduino circuit (13) that controls the peristaltic pump, Raspberry Pi (12). (C) Recorder of output signals by Raspberry Pi. The functionalised structure with seeded cells is represented in red, and the same structure without cells is represented in black. (D) Pressure amplitude of the P-wave exciting transducer vs voltage amplitude. (E) Flow calibration results, flow vs voltage amplitude of the peristaltic pump. (F) 2D fluid dynamic model in FEM to evaluate the incidence flow speed (a and c) on fibres of the structure and their corresponding shear stresses (b and d) depending on the flow. Two flows are represented: 0.5 ml·min-1 and 0.8 ml·min-1.

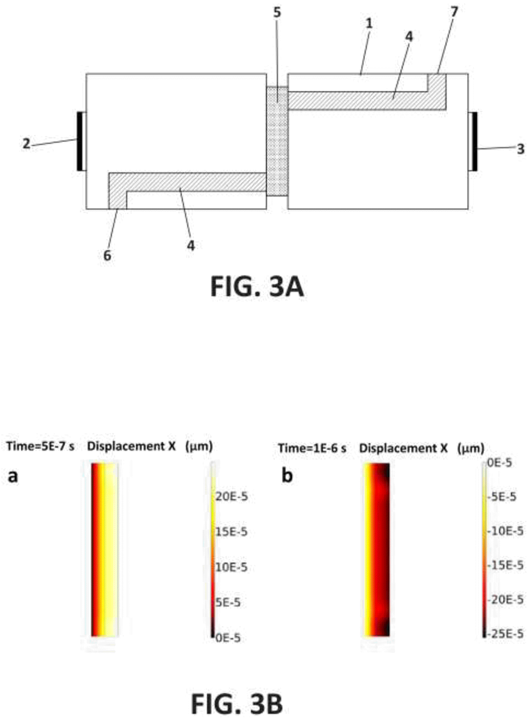

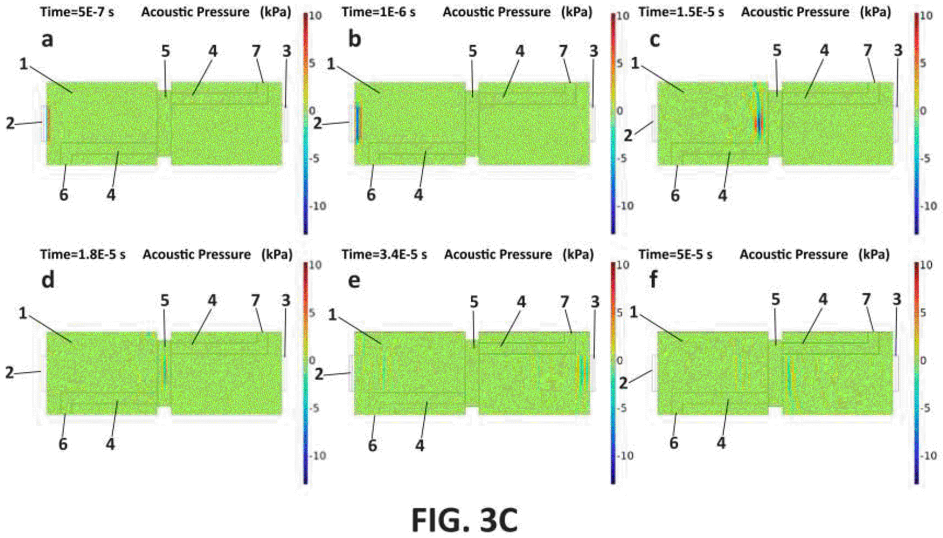

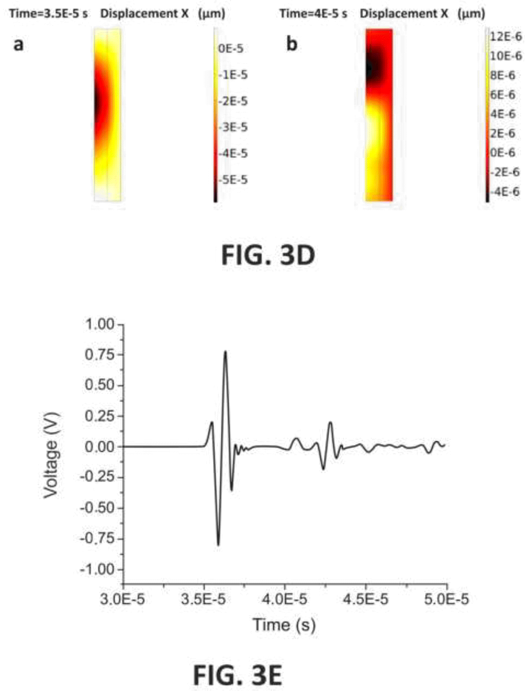

Figure 3. FEM model of P-wave propagation phenomena. (A) 2D representation of the different elements of the bioreactor: culture chamber (1), transmitting transducer (2) arranged opposite the receiving transducer (3), fluid circuit (4) intended to pass through the structure (5) that accommodates the cell source, fluid system inlet (6), fluid system outlet (7). (B) Thermal image of the "u" component of the displacement of the transmitting ultrasound transducer, (i.e., component X) at t = 0.5 $\mu$s (a) and t = 1 $\mu$s (b). (C) P-wave propagation at different times: (a) t = 0.5 $\mu$s, (b) t = 1 $\mu$s, (c) t = 15 $\mu$s, (d) t = 18 $\mu$s, (e) t = 34 $\mu$s, and (f) t = 50 $\mu$s. (D) Thermal image of the "u" component (i.e., component X) of the displacement of the receiving ultrasound transducer at t = 35 $\mu$s (a) and t = 40 $\mu$s. (b). (E) Final electrical modelling signal obtained from the FEM model.

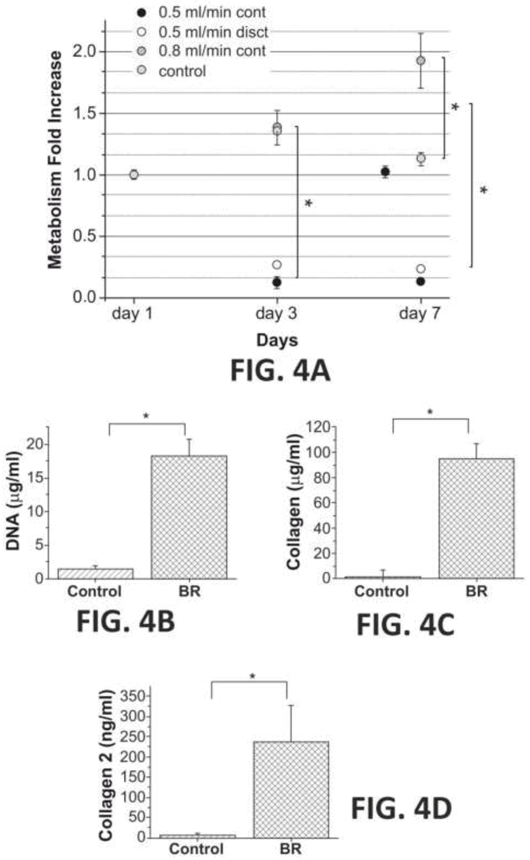

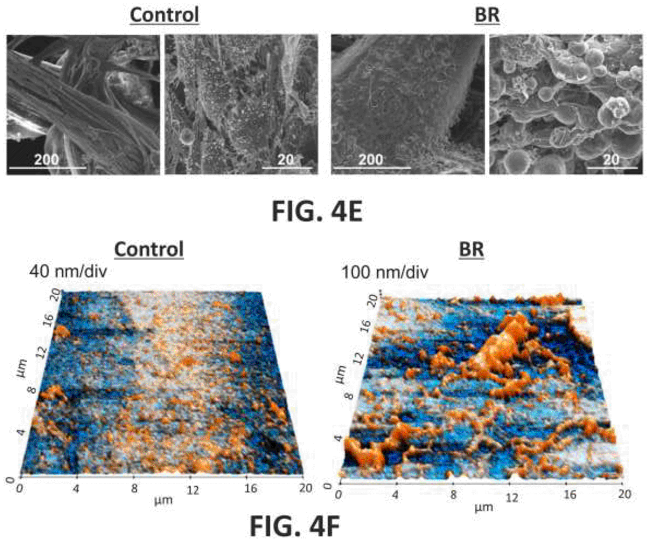

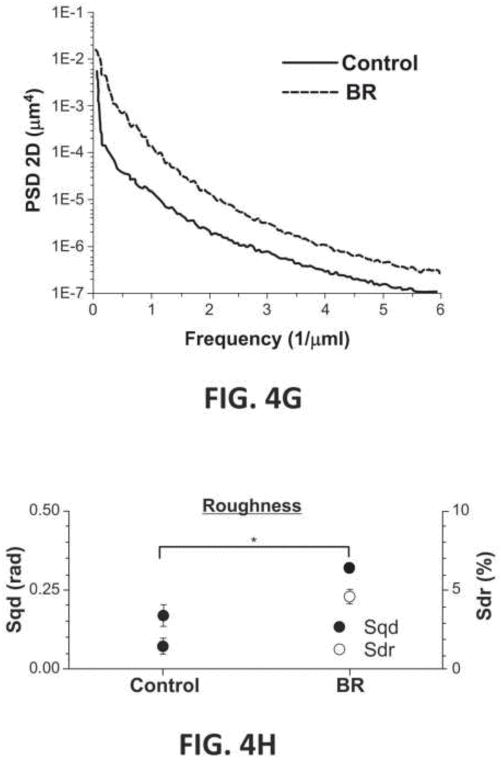

Figure 4. (A) Alamar Blue assay to detect metabolic activity in PBA-functionalised bTPUe structures after seeding IPFP-MSCs with different flows and control. The values were normalised with respect to day 1. (B) Quantification of DNA on day 7 of the control experiment and bioreactor (BR) 0.8 ml·min-1. (C) General quantification of collagen with Sirius Red assay of the control sample and BR sample (0.8 ml·min-1). (D) Quantification of type II collagen using the Elisa kit for both control and BR samples (0.8 ml·min-1). (E) SEM images on day 7 of both structures, control without cells and BR with cells, the scale bars are expressed in microns. (* means p-value < 0.001; cont. = continuous perfusion flow, disct. = discrete perfusion flow). (F) Contrast-enhanced images of a region of interest (ROI) from both conditions. (G) Power spectral density (PSD) profile of the average of three samples. (H) Values of the root mean square gradient (Sqd) and the developed interfacial area ratio (Sdr) that establish a statistical difference (p < 0.001) between the control samples and the BR condition.

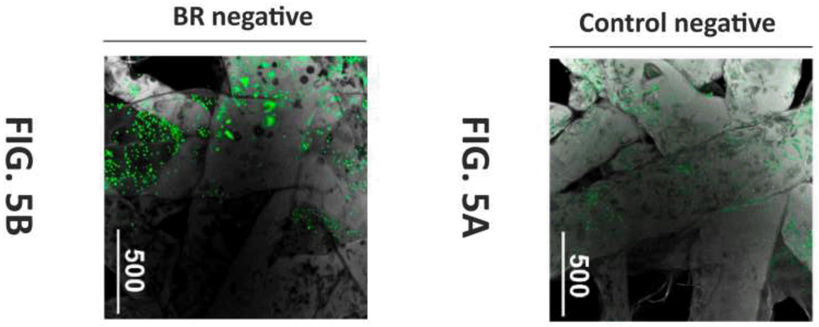

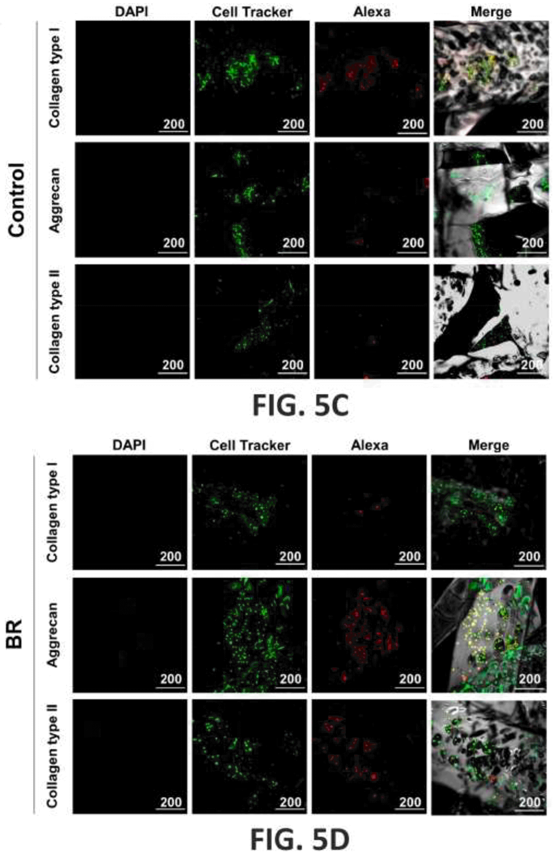

Figure 5. Immunofluorescence imaging. Imaging without a primary antibody (i.e., negative) of the control sample (A) and the bioreactor->BR sample (B). (C) Control samples and BR samples (D) with a type II collagen primary antibody + 2nd Ab, and a primary aggrecan antibody + 2nd Ab. Cell Tracker™ is represented in green. The secondary Ab (Alexa 647 nm) is shown in red. The DAPI marker is represented in blue.

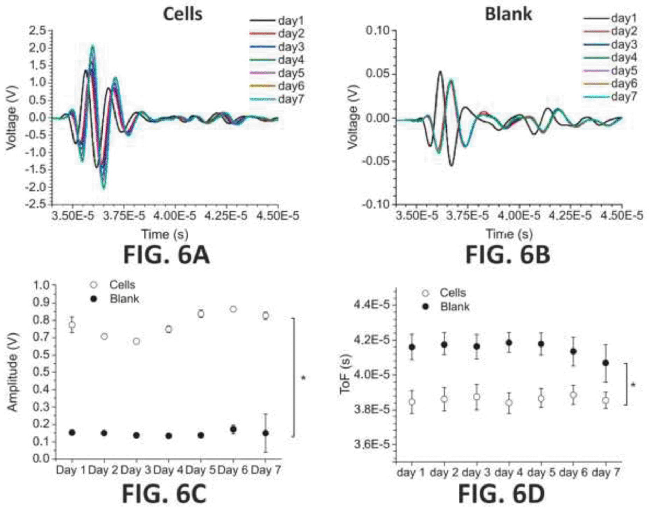

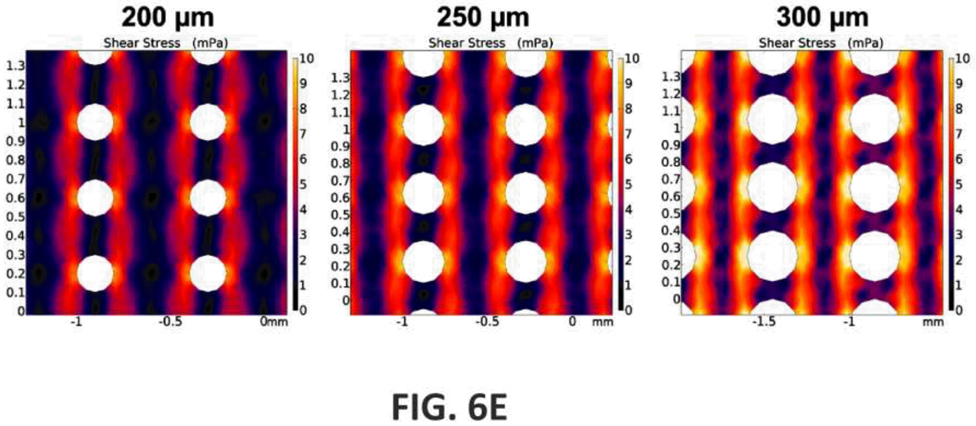

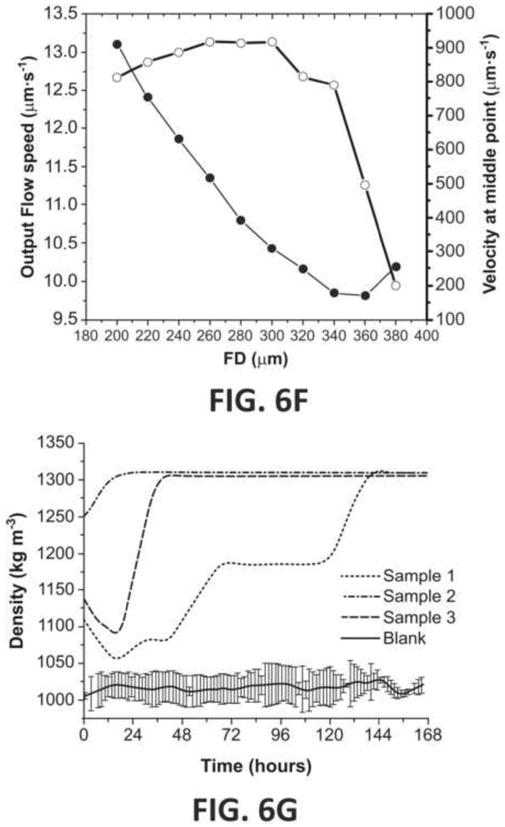

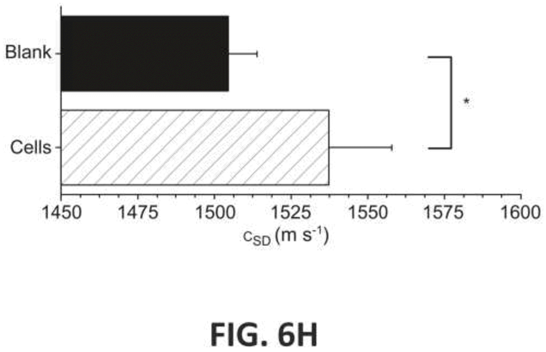

Figure 6. (A) Arbitrary P-wave signals obtained from the cell-containing structures during experimentation in the bioreactor BR. (B) Arbitrary P-wave signals obtained from the blank structures (without cells) during BR experimentation. (C) Amplitude evolution curve of medium amplitude signals. (D) Time-of-flight (ToF) curves. (E) 2D thermal images of the fluid shear stress (CFD) model using different fibre diameters of the structure. (F) Output flow speed of the BR versus the fibre diameter (FD) of the structure in black; and the instantaneous velocity at the middle point (X = 0, Y = 0) within the structure domain. (G) Density evolution over time of BR cell content in samples of structures with cells and blank structures (without cells) that are obtained by cross-correlation empirical signals with synthetic signals. (H) Speed of sound of the structure domain (cSD) (structure + water) obtained by cross-correlation. (*: p-value < 0.001).

## PREFERRED EMBODIMENT OF THE INVENTION

The bioreactor of the invention, compared to static cultures, enhances chondrogenesis through low shear stresses by means of fluid perfusion. The bioreactor boosts the growth of stem cells seeded on PBA-functionalised bTPUe structures and induces increased production of ECM with a high content of type II collagen.

The use of SEM microscopy (scanning electron microscopy) and confocal images provide evidence that the structures with cells stimulated by the bioreactor increased both the cellular presence and the extracellular matrix (ECM) of the biological tissue produced, cartilage (AC).

An FEM finite element computational model was performed to evaluate and understand the effective effect of a pressure wave exerted by ultrasound on the culture. To do so, a set of synthetic signals was obtained through the inverse problem, where different parameters of the model were swept.

The use of an FEM to analyse fluid dynamics reveals the need not only to regulate the initial flow, but also to modify it over time to accommodate tissue growth.

The invention also proposes a method that uses the bioreactor of the invention together with the FEM and allows the increase in tissue density over time to be independently tracked and the speed of sound in the tissue to be determined using an inverse characterisation of problems through the FEM model. In other words, the unknown initial parameters of the problem to be treated are obtained from different solutions. As a result of the fact that both metrics are closely associated with an increase in material stiffness and collagen content, it makes it possible to infer that the method of the invention is capable of monitoring tissue growth over time.

Therefore, the bioreactor of the invention and the associated method have the capacity to induce the production of biological tissue through structures that accommodate a cell source, as well as the capacity to measure the growth and maturation of tissue in real time.

The bioreactor comprises a culture chamber (1) intended to accommodate a three-dimensional structure (5), the geometry of which, in this case, is a central ring with a diameter of 24 mm and a total length of 45 mm, of which 5 mm correspond to the size of the structure (5) and the rest correspond to relief distances, 20 mm per side, to avoid the near-field effect of the ultrasound signal. The three-dimensional structure (5) is intended to promote cell culture, such that a cell source of mesenchymal stem cells (MSC) from the infrapatellar fat pad (IPFP-MSC) is introduced.

The bioreactor also comprises an inlet (6) and an outlet (7). In this case, the inlet (6) and the outlet (7) are cylinders made of highly transparent thermoplastic polymer (PMMA) rods with a diameter of 30 mm and a length of 40 mm and that are perforated to form fluid channels (Figure 1). The length of the cylinders allows the presence of a fully developed laminar flow. When a fluid enters a pipe, the minimum length to generate a fully developed laminar flow must fulfil: $\frac{L}{D} = 4.4 Re^{1/6}$.

The fluid used in this case is a culture medium [Dulbecco's Modified Eagle Medium (DMEM)] rich in glucose, 10% foetal bovine serum (FBS) that is Newtonian ($\rho = 1009\ kg \cdot m^{-3}$; $\mu = 0.93\ mPa \cdot s$) with Re = 4.6. Therefore, L to fulfil the foregoing conditions is 22.7 mm.

The inlet (6) and outlet (7) cylinders have a length of 35 mm, greater than the minimum required. Furthermore, the length of the near field is (N): $N = \dfrac{D^2 f}{4c} = 15.4 \; mm$ , with the speed of longitudinal sound of PMMA equal to $c$ = 2730 $m \cdot s^{-1}$.

The bioreactor further comprises a transmitting transducer (2) and a receiving transducer (3) of ultrasonic waves. The transmitting transducer (2) can be coupled using a hollow cylinder with a restriction ring and a bTPUe pad that exerts pressure to acquire an accurate signal (Figure 1D).

Figure 1E schematically represents the electronic assembly of the bioreactor (BR). The MSO6054A oscilloscope has been programmed to capture each signal between 30 μs and 50 μs; said time period was manually set the first time that the time of flight (TOF) of the signals from the structure (5) was displayed (approximately 35 μs). The signal capture interval was set to 5 minutes to save memory and allow enough time for the transfer of signals between the oscilloscope (11) and the Raspberry Pi (12) and the capture signals were the average of 200 signals measured with 8-bit resolution. Furthermore, to ensure good communication between Arduino, the microcontroller that regulates the pump (8) (programmed in C++), and the Raspberry Pi (12), which was the applied computing unit (Python), a parser library was applied (ParserLib by LuisLlamas).

Figures 2A and 2B show the entire bioreactor assembly together with an electronic circuit that regulates the peristaltic pump (8). All components must be previously sterilised with Biozidal ZF and 70% ethanol, followed by UV light turned on inside the laminar hood. Then, the bioreactor is coupled and begins to operate with flows no greater than 0.5 $mLmin^{-1}$ to ensure there is no cell damage.

Figures 2C to 2E represent experimental parameters obtained from the bioreactor. Figure 2C shows two arbitrary ultrasound signals recorded on the Raspberry Pi (12), a signal represents the structure (5) with cells for its culture, while the other signal is a control signal, in other words, the structure without cells. The amplitude of the signal with cells is almost double that of the control signal and the bounces closer to the main signal (approx. 40-45 μs) are more attenuated in the signal with cells.

Figure 2D represents the incident pressure exerted by the waves produced by the transmitting transducer (2). In this case, although the wave generator had a limiting output amplitude of 20 V, to verify the linear response of the exerted pressure with respect to the input excitation voltage, a curve from 0 to 50 V is shown. The pressure obtained was extracted via the wave hydrophone calibration formula from the manufacturer's data sheet: $P = 0.35 \, V_m / 10^{-\frac{254}{20}+6}$ , applying a linear regression with $R^2$ = 0.99 with P = 72.6 · $V_m$ + 43.8. For the experimental procedures, an excitation voltage $V_{pp}$ = 20 $V$ was selected, where an acoustic pressure of 1509 Pa was obtained. Regarding the flow, a weighing protocol was established, where the voltage varied from 5V, minimum operating voltage of the pump (8), to 24V, maximum operating voltage, in steps of 0.5V. The extracted results express that to obtain flows lower than 1 $mL \cdot min^{-1}$ , it was only necessary to apply 12V. In this regard, with such results and to increase the control resolution, taking into account that Arduino has an 8-bit resolution for power modulated (PWM) outputs, the power supply to the pump (8) is limited to 12V.

Figures 2Fa to 2Fd show a microfluidic model through the fibres of the structure (5) with two different flows. The structure (5) is represented as a matrix of circles, where each circle has a diameter of 200 μm and the circles are separated by 600 μm due to the printing layer pattern. The size of the mesh element was adapted to 100 μm since it is ½ of the fibre diameter. The average cell density ($\rho$ = 1.009 g·cm$^{-3}$) and the dynamic viscosity ($\mu$ = 0.93 mPa·s) are known from the state of the art. The Reynolds number is calculated as:

$$Re = \frac{\rho u D_h}{\mu} = 4.6 \ll 2300$$

where u is the inlet flow speed, where u = 106. 1 μm·s$^{-1}$ to 0.8 Ml·min$^{-1}$; and u = 66. 31 μm·s$^{-1}$ to 0.5 Ml·min$^{-1}$, and $D_h$ is the height of the inlet diameter, which was 4 mm. The Re obtained is less than 2300, which is considered the upper limit for laminar flows, while turbulent flows develop when the Re number exceeds 2900.

According to the results of the model, the flow obtained by weighing can be translated into real flow speeds incident on cells: a) 0.5 $mLmin^{-1}$ corresponds to flows lower than 800 $\mu m \cdot s^{-1}$ and b) 0.8 $mLmin^{-1}$ corresponds to flows lower than 1000 $\mu m \cdot s^{-1}$. It is important to note that although the flow speed is greater between the fibres, close to the fibre surface (i.e., the location of the cell), the flow rate decreases to below half the maximum value (Figure 2F). The maximum shear ratios can be extracted from the model, which is 10 mPa at 0.5 $mLmin^{-1}$ and 16 mPa at 0.5 $mLmin^{-1}$.

Furthermore, as part of the present invention, a complex 2D model has been developed by applying a multiphysics FEM to understand the mechanical behaviour of the bioreactor ultrasound system. Figure 3 shows the representation of the model and the physics behind its behaviour. Figure 3A represents the visualisation of the XY plane of the geometry, where the modelled plane corresponds to a cross section in the centre of the bioreactor. This plane was selected in both models (P-wave propagation and fluid dynamic model) because there is no axial symmetry for the flow channels.

To validate the developed model, the bioreactor was assembled without any structure therein, filled with water and with a solid polylactic acid (PLA) disc. In previous literature, both the density and the speed of sound have been obtained. The speed of sound of PMMA was empirically calculated at 2630 m·s⁻¹. A correlation was obtained, comparing synthetic signals with experimental signals, over 80%.

The signal originated in the transmitting transducer (2) as a mechanical wave (P). Figure 3B shows the mechanical displacement in the X direction, while the displacement in Y was negligible. The total displacement ranges from -25.10⁻⁵ $\mu$m (3B.a), at t=T$_0$/2, to 25·10⁻⁵ $\mu$m (3B.b), at t=T$_0$. After that, the P-wave propagates through the acoustic pressure module (Figure 3C), where the dependent variables were material densities ($\rho$) and material speeds of sound

$$(c_s): \frac{1}{\rho c^2} \frac{\partial^2 p_t}{\partial t^2} + \nabla \cdot (-\frac{1}{\rho} (\nabla p_t - q_d )) = Q_m$$ , where $Q_m$ is the monopole domain source, $q_d$ Is the dipole domain

source, and $p_t = p + p_b$ with $p_b$ is the background pressure field. It must be taken into account that the internal impedance is a limit condition that depends on $\rho$ and $c_s$. Therefore, signal attenuation will depend on the density and the speed of sound of the domain of the structure (5). Therefore, for the outer limits representing the walls of the bioreactor

$$-n \cdot (-\frac{1}{\rho} (\nabla p_t - q_d )) = 0$$ .

In Figure 3C-a, which corresponds to T$_0$/2, the P-wave is partially developed; in Figure 3C-b, it is fully developed and shows that the P-wave contacts the domain of the structure (5) at t=15 $\mu$s (3C-c); at t=18 $\mu$s (3C.d), it is in the centre of the domain of the structure (5), which implies that the main wave travels through the domain of the structure (5) in approximately 6 $\mu$s. Next, at 34 $\mu$s, the main wave reaches the receiving transducer (3C-e), and at t=50 $\mu$s, there are only bounces obtained from different boundary conditions (3C-f).

As was done in the transmitting transducer (2), Figure 3D shows the displacement in the X direction of the mechanical P-wave over the domains of the receiving transducer. It is observed that at t=35 $\mu$s, the P-wave is fully developed and at t=40 $\mu$s, wave bounces are transmitted. Lastly, Figure 3E represents an ultrasound wave obtained by the proposed model as a floating potential of the receiving transducer.

A 2D model was chosen because it reduces computational time, memory requirements and representation complexity. All the foregoing parameters are essential to calculate studies of inverse problems where parametric sweeps are performed. Unlike the previous model, which describes microfluidic flow, in this model, porosity has not been modelled because the wavelength of the ultrasound wave (P) through the domain of the structure (5) is much greater than the pore

size: $\lambda_{SD} = \frac{c_{SD}}{f_0}, c_{SD} > 1480 \, m \cdot s^{-1}, f_0 = 1 \, MHz, \lambda_{SD} > 1.48 \, mm \text{ and } \mathrm{PS} < 400 \, \mu m$ *and* PS < 400

$\mu$m , where SD is the domain of the structure (5) and PS is the pore size. The size of the mesh element was selected by parameter sweep to verify convergence of the model. Therefore, it was established that an element size smaller than 400 $\mu$m was sufficient to acquire a stable signal. In Supplementary Figure 3G-3J, no significant variation on amplitudes at mesh sizes smaller than 400 $\mu$m are shown. The rest of the parameters (*i. e.*, $c_{PMMA}$, $c_{H2O}$, $\rho_{PMMA}$, $\rho_{H2O}$) were reconstructed using the inverse problem.

The Alamar Blue reagent was used to detect metabolic activity in the PBA-functionalised bTPUe structure (5) after seeding IPFP-MSC cells. In order to evaluate cell proliferation and viability, different flow regimes and flows were carried out (Figure 4A).

IPFP-MSC cells were isolated and grown from patients with OA until stage 3 - 5. IPFP-MSC cells were identified using standards recommended by the International Society for Cellular Therapy (ISCT). Next, 10⁶ cells·Ml⁻¹ were seeded on structures and incubated for 4 h at 37°C before receiving new media [(DMEM Glutamax, 10% FBS, 1% penicillin/-streptomycin (P/S)]. The structures with cells were cultured for 1 week in a 6-well plate to ensure proper integration between the cells and the structures. Then, the structures were introduced into the bioreactor for another week, while other cell-laden control structures were kept in the well plate that week. The metabolic activity of all the structures was tracked on days 1, 3 and 7 of the experimental time.

The results obtained indicated a different response in cellular metabolism depending on the flow rates (Figure 4A). Following the recommendations of the literature, a continuous flow of 0.5 ml·min⁻¹ was tested with negative results. Next, a discrete form (1 h every 6 h) of pre-flow was applied to reduce the exposure stress on the cells; negative results were once again obtained with a dramatic reduction in cellular metabolism on day 3. Furthermore, once the flow was increased to 0.8 ml·min⁻¹, metabolism increased similarly with respect to the control structures. In fact, on day 7 in the bioreactor with 0.8 ml·min⁻¹, metabolism continued its growth in comparison to the rest of the conditions, reaching almost double the rate of day 1. Consequently, the following experiments were performed only with a flow of 0.8 ml·min⁻¹.

Furthermore, DNA quantification was carried out for both the bioreactor structures and the controls to see if the changes observed in metabolic activity were associated with differences in cell density. The results presented in Figure 4B show a clear difference between the bioreactor samples and the control samples with respect to the amount of DNA, with a significant increase in the bioreactor structures (p < 0.001). In relation to ECM synthesis, both the general quantification of collagen based on the Sirius Red dye and the quantification of type II collagen were carried out. Figure 4C shows similar results with respect to collagen content, a much greater presence of collagen in bioreactor samples (p < 0.001). Furthermore, a much higher concentration of type II collagen was obtained (Figure 4D) in the bioreactor samples compared to the control sample (p-value < 0.001).

To verify the increase in the amount of ECM in 3D structures included in the bioreactor, scanning electron microscopy (SEM) and immunofluorescence techniques were applied. In the SEM results (Figure 4E), two particular facts were observed: i) the fibres of the structure (5) in both samples (control structure and bioreactor structure) were covered by cells; however, while there were empty spaces between the cells in the control structure (5), the fibres of the structure in the samples incubated in the bioreactor were completely covered by both cells and ECM. Likewise, the presence of thicker fibres can be seen. ii) Regarding cell morphology, the control samples exhibited a different shape of the IPFP-MSC cells from the bioreactor samples where the cells showed a more circular shape. However, in both cases, cell viability and adequate cell-biomaterial interaction were demonstrated.

In addition to the SEM microscopy images, atomic force microscopy (AFM) was applied to characterise the surface roughness of the fibres of the structure (5) between the cells. Contrast-enhanced images of a region of interest (ROI) from both conditions can be seen in Figure 4F. The results express an observable increase in roughness in terms of quantity and grain size in the bioreactor structures with respect to the control samples. Moreover, the presence of some fibrils (collagen) is evident in cases in which the bioreactor has been used. To quantify the degree of exchange in roughness, the average power spectral density (PSD) profile between three samples was calculated in Figure 4G. The curves show a higher signal power in the bioreactor samples than in the control samples, which implies the presence of higher structures attached to the surface of the structure, as previously observed in Figure 4F. Lastly, typical roughness parameters such as the root mean square gradient (Sqd) and the developed interfacial area ratio (Sdr) were also calculated, which establish a statistical difference (p < 0.001) between the control samples and the bioreactor samples, which implies that the roughness of the structure increased with the exerted perfusion flow (Figure 4H).

For immunofluorescence imaging, Figures 5A and 5B correspond to images with the absence of primary antibody (negative) to verify whether the secondary antibody exhibited non-corresponding interactions. To avoid autofluorescence of the structure (5), Alexa Deep Red was selected as a secondary antibody; therefore, each ECM protein was represented separately (Figure 5C and 5D). The results clearly expressed an abundant presence of all the common ECM proteins of the AC and high cell viability in the bioreactor samples: type I collagen, type II collagen and aggrecan. The bioreactor structures exhibited a much higher expression of type II collagen and aggrecan, with low amounts of type I collagen. In contrast, the control structures exhibited a much lower amount of cell content, without the presence of aggrecan proteins and a much higher content of type I collagen. In conclusion, based on SEM microscopy and immunofluorescence quantification assays, the chondrogenic induction potential of the bioreactor on IPFP-MSCs cells was demonstrated.

Since ultrasound is a valuable non-invasive characterisation method, low-pulse ultrasound (LIPUS; 1 MHz) was used in this research to study the mechanical evolution of the biomanufactured tissue over a week inside the bioreactor. To take out any information from the background, cell-free structures (blank) have also been analysed with ultrasound and responding to the same conditions as the *ex vivo* experiment. Figures 6A and 6B show arbitrary signals from each experimental day for structures seeded with cells and blank (without cells), respectively. In both cases, a decrease in the voltage $V_p$ was observed after 24 h of treatment with a signal delay. The presence of this phenomenon in both conditions implies non-cell-dependent behaviour. Furthermore, it can be seen how $V_p$ increased from day 2 to day 7 in the cell-laden structures (from 1.3 V to 2.1 V), while no substantial variation was shown in blank structures (0.04 V to 0.043 V).

Figure 6C represents the evolution of signal amplitudes over time. In the blank structures, no significant difference was observed throughout a week of experimentation. Even though the decrease in amplitude from day 1 to day 3 was observed in both cases, there was a noticeable increase in amplitude only in cell-laden structures. Another important consideration, which can also be seen in the previous Figures 6A, 6B and 6C, is the significant difference of the amplitude value on all days (p < 0.001). The same difference was evident with respect to the calculated time-of-flight (ToF) signal (Figure 6D), where the signals from blank structures were slightly slower than those from cell-laden structures (p < 0.001). In summary, the results observed in Figures 6C and 6D imply that the received ultrasound signals expressed considerable differences when the cells were seeded on the structures.

The raw data recorded do not provide a complete prediction of the ECM synthesis rate. As explained earlier, computational models were developed to describe both fluid dynamics and wave propagation phenomena (P). Thanks to these models, it can be estimated how the fluid could affect the cells if the fibres of the structure (5) become larger over time. The idea behind the foregoing argument is expressed in Figure 6E, where three different fibre diameters are proposed: initial fibre diameter of 200 $\mu$m, an intermediate fibre diameter of 250 $\mu$m and an estimated final diameter of 300 $\mu$m (which

is approximately that obtained from the SEM microscopy images). It is confirmed that the shear stresses ($\tau = \mu \dfrac{\partial u}{\partial y}$, *with*

$\mu$ *being the fluid dynamic viscosity*.) will increase directly proportional to the diameter of the fibre, in particular, there is a range of initial 4-7 mPa to a final 8-10 mPa. However, more important is the presence of high shear stresses in the orthogonal direction of the fluid flow once the fibre diameter exceeds 250 $\mu$m.

Figure 6F shows the bioreactor output flow speed, which is very similar to Figure 6E in that a boundary probe recorded data such as instantaneous flow speed (at the axial point of origin) and output flow speed magnitude. The foregoing magnitudes were compared to an estimated increase in the fibre diameters (FD) of the structure. It is shown that both magnitudes are connected to each other and exhibit different patterns. The output speed decreased virtually linearly for fibre diameters of 360 $\mu$m, although the immediate speed increased from 200 to 320 $\mu$m. For fibre diameters greater than 320 $\mu$m, the output flow speed began to decrease slightly, but at fibre diameters of 360 $\mu$m, there was a sharp drop followed by a change in trend. This observation implies that when the fibre diameter exceeds 340 $\mu$m, the permeability of the structure decreases, and the fluid channel cannot cross the structure, but rather it passes around its walls.

More interesting is the case of the ultrasound model and the wave propagation model (P), where the mechanical behaviour of three different materials (PMMA, water and bTPUe) must be considered. Therefore, the final electrical signal that represents the ultrasonic pulse involves all those parameters, which makes extracting information a very complex task. Once again, the computer model focused on the reconstructive parameters that had the most variability over time. In this regard, for the wave propagation (P), the equation for longitudinal wave propagation speed can be highlighted:

$$c_p = \sqrt{\frac{E(1-\nu)}{\rho(1+\nu)(1-2\nu)}} \;\rightarrow\; c_p = \sqrt{\frac{K}{\rho}}$$ . The previous equation estimates that the final signal obtained by the receiving transducers will mainly depend on the speed of sound in the structure and its density.

With this in mind, a set of signals that varied the speed of sound in the structure domain ($c_{SD}$) from 1300 m·s$^{-1}$ to 1600 m·s$^{-1}$ and the density of the structure domain ($\rho_{SD}$) from 1000 kg·m$^{-3}$ to 1400 kg·m$^{-3}$ was synthetically created. Then, empirical signals obtained from *ex vivo* experiments in the bioreactor were compared with said set of signals. Next, each empirical signal was cross-correlated, $(f \star g)(x) \stackrel{\text{def}}{=} \sum_j f_j^* g_{i+j}$ , with each synthetic signal generated with the computational model. Each synthetic signal was labelled with its corresponding density $\rho_{SD}$ and $c_{SD}$, then, it was estimated that the maximum cross-correlation (and always >80%) were the most acceptable values of density and speed of sound.

Figures 6G and 6H show the extracted parameters ($\rho_{SD}$ and $c_{SD}$). Figure 6G indicates two pieces of evidence: i) no significant variation in density was observed in blank structures; ii) there was an increase in densities in the structure domain over time in structures seeded with cells. A non-averaged curve was calculated on cell-seeded structures to show the capacity to record different growth patterns. Furthermore, Figure 6H shows the speeds of sound of both the cell-seeded structures and blank structures. The average values are represented due to much smaller variation (SD < 1%) of this parameter compared to density. The figure again shows a significant difference between the blank structures and the cell-containing structures (p < 0.001), where the speed of sound ($c_{SD}$) of the cell-laden structures is greater. It is important to consider that the values $c_{TPU} = 1740\, m \cdot s^{-1}$ and $c_{H_2O} = 1480\, m \cdot s^{-1}$ from the literature are quite close to what was obtained in this experiment and model. Furthermore, the pore ratio of the printed structures was 0.3, where $c_{SD} = 0.3 \cdot c_{TPU} + 0.7 \cdot c_{H_2O} = 1558\, m \cdot s^{-1}$. From this, it can be deduced that the results obtained from the model are highly precise, and that a greater speed of sound implies less water content in the structure domain.

The model presented shows not only the flow magnitudes but also the shear stresses exerted, obtained thanks to the results of FEM microscopy (Figure 2F). Furthermore, the filling of the bioreactor where no significant turbulence appears was modelled using two-phase fluid dynamics. Plan representations make it possible to identify the regions of greatest stress, which implies that not all areas of the structure (5) will experience the same load regime. It is a small region near the corners of the inlet (6) and outlet (7) channels that has higher stresses, while the rest of the structure (5) experiences a smaller stimulation. As a result, the presence of homogeneous (i.e., isotropic) stimulation throughout the structure (5) of the bioreactor cannot be assumed, as considered in the state of the art.

Furthermore, the FEM analysis also improves overall understanding due to its possibility of simulating evolutions. In Figure 6E, a hypothesis of increasing fibre diameter over time was assumed; looking at the graphs, it is obvious that the shear stresses increase with the diameter of the fibres of the structure (5) (from 6 mPa to 10 mPa).

It is important to precisely control the evolution of these shear stresses because they can cause cell damage. Furthermore, if sufficient perfusion flow is not exerted, the risk of poor nutrient transport will ensure cell death, as possibly occurs at flow rates of 0.5 ml·min$^{-1}$ (see Figure 4A). By extrapolating these observations and comparing them with the simulation obtained in Figure 6F, it can be concluded that as the fibre diameter increases, the flow through the structure (5) (tissue) decreases and, consequently, nutritional intake decreases. This consideration corresponds to the results obtained by the cross-correlation model of the experimental waves (P), where a density limit ($\rho$) was observed.

In relation to the biological assays, where the metabolism of the structures within the bioreactor was analysed along with their chondrogenic potential to generate cartilage-like structures, the following assertions can be made. Once the inlet flow was optimised, in other words, continuous flow of 0.8 ml·min$^{-1}$, it was observed how cellular metabolism increased in the structures with cells before it did so in the control structures, reaching almost 200% growth. Likewise, a reduction in cellular metabolism occurred in the control structures from day 3 to day 5.

Cellular metabolism is highly determined by nutrient transport and perfusion flows, improving the intake of metabolites and promoting an increase in metabolism. Therefore, the results show that cellular metabolism increased, but also that the cell content was significantly higher, indicating that the cells expanded under the bioreactor stimulation protocol. Moreover, the general collagen quantification assay together with the type II collagen values obtained demonstrate the chondrogenic potential of the bioreactor structure. It is thus observed that there is a presence of collagen and a greater production of type II collagen. These results indicate that the bioreactor of the invention dramatically improves the chondrogenic potential of the cell-laden structures.

Imaging techniques (SEM microscopy and immunofluorescence) corroborated the presence of cells attached to the fibres of the structure in both the blank samples (control) and the bioreactor samples. In the control samples, it was possible to distinguish how the MSCs retained their elongate and flat morphology and the presence of some surrounding ECM cells was also observed. By contrast, in the bioreactor samples, the morphology of the MSCs was lost, exhibiting a rounder shape embedded in a matrix with some cilium that is typical of chondrocyte-like morphology. Furthermore, the ECM present in the bioreactor samples was considerably higher compared to the control samples and the fibres of the structure that appear to be completely covered by said matrix. The results obtained by SEM microscopy were confirmed by AFM microscopy analysis, showing that greater roughness was obtained in the bioreactor samples and the presence of ECM was found to be similar to that of AC along with thicker fibres, which are a marker of healthy AC.

Lastly, immunofluorescence was performed to detect typical chondrogenic ECM proteins. The presence of both type I collagen, type II collagen and aggrecan in bioreactor and control samples was observed. For the controls, much lower amounts were observed for type II collagen and aggrecan and an increased expression of type I collagen was detected. In contrast, the bioreactor samples showed higher levels of viable cells attached to the fibres, a lower expression of type I collagen and higher amounts of type II collagen and aggrecan, which implies chondrogenesis.

Moreover, immunofluorescence imaging showed how these proteins were distributed among cells along the fibres of the bioreactor structure, while in the control samples they concentrate on the fibre interlacing. In previous literature, it was found how MSCs express type I collagen and hyaluronan, where an early state of pre-cartilaginous condensation is the digestion of the type I collagen matrix and the expression of N-cadherin, tenascin-C and other adhesion proteins regulated by TFG-$\beta$, Wnt/$\beta$-catenin and Sox9 signalling pathways. Lastly, the pre-cartilaginous ECM becomes ECM of the AC rich in type II collagen and aggrecans. Furthermore, the expression of Sox9 and Col2a1 is correlated in chondrogenesis with the synthesis of type II collagen. In this model, the increase in the expression levels of type II collagen and the overexpression of said genes are quantified and observed after stimulation of the bioreactor.

All these results indicate that the PBA-functionalised structures located in the culture chamber (1) of the bioreactor improve cell proliferation and chondrogenesis, reducing differentiation times compared to the state of the art and without the need for an additional chondrogenic medium.

The present invention also relates to the development of a method for analysing cell growth and differentiation in real time. The objective is to obtain a self-sufficient system that can predict and quantify cell viability of the biomanufactured tissue before being implanted.

The in situ sensors are connected directly to the bioreactor (invasive), while the at-line systems depend on a sample that is drawn and evaluated on the outside. The at-line measurement of these sensors is carried out when data are captured continuously and the response time of the sensor signal is short compared to the dynamics of the process. Any other measurement is considered off-line, which implies a time delay due to biological activities. Here, focus is placed on non-invasive at-line systems since they prevent contamination, and which include: optical chemo-sensors, spectroscopic sensors, impedance spectroscopy sensors, ultrasonic sensors and wireless free-floating sensors.

The bioreactor of the invention is the first *ex vivo* system to quantify chondrogenesis of the structure (5) located therein in real time. In this bioreactor, an excitatory wave (P) is applied using ultrasound transducers to record the pulse emitted after passing through the structure (5). Furthermore, an FEM model that predicts possible physical events is generated. In this case, the structure domain (SD) was considered as a homogeneous domain formed by the mixture of water and bTPUe (with attached cells and ECM). This approach is based on the link between the size of the FEM element to be examined and the excitation wavelength, where their ratio is crucial to derive information from the received wave in the field of non-destructive evaluation using ultrasonic waves. When the wavelength is greater than the size of the element to be examined, data are obtained from the environment, which is considered homogeneous; however, when the wavelength is smaller than the size of the element to be characterised, the mechanical data of the element in question are recognised in the received wave. The structure (5) has been treated as a homogeneous medium without modelling it, since the excitation wavelength (1.5 mm) is larger than the pore size (400 $\mu$m) or the fibre diameter (200 $\mu$m).

In this case, the wave interaction, together with the assumption that the structure (5) is a homogeneous medium, results in the validation of the wave information obtained. Therefore, depending on the mechanical characteristics of the bioprinting material of the structure (5) and its printing percentage, as well as the mechanical properties reconstructed with the culture model, the wave speed is estimated to correspond to the empirically recorded time of flight (ToF). This estimate can be made in the calibration phase of the system knowing the physical properties of the material of the structure (5), its acoustic dissipation and its density. Furthermore, in the case of the structure (5) that accommodates the cell source, after generating the model, a database of simulated waves can be obtained with which the speed of sound and density are obtained. With the obtained speed of sound, the time of flight can be estimated and compared with the time of flight obtained empirically.

The results extracted from the assays in the bioreactor show that the amplitude of the cell-laden structures was greater than in the blank structures, which implies that the stiffness of the structures with cells was greater. A shared characteristic between the cell-laden structure samples and the blank samples was the decrease in amplitude from day 1 to day 3, which indicates an external cause of such phenomena and is probably due to a plastic deformation of the materials that exert the contact stress of the transducers.

Furthermore, the time of flight (ToF) value is represented as an unsuitable parameter for the prediction of tissue expansion, since its variations are much smaller than the rates of cell proliferation and ECM synthesis. More interesting is the absence of considerable near bounces in the study samples when compared to blank structures, which implies smaller pore sizes. In this regard, the empirical signals obtained in this case express a clear differentiation between measuring the structure with cells attached to its surface and measuring the structure without attached cells (blank). Therefore, the application of an FEM to induce big data analysis tools such as cross-correlation of signals are precise tools to expand the information of the extracted results and, in this case, to transform a qualitative approach into a quantitative one.

Furthermore, given that a clear increase in the density of the structure domain was obtained over time in the bioreactor and this was not due to any type of bTPUe degradation after 14 days, this result can only be explained by ECM synthesis and cell proliferation. Consequently, it is verified that the method of the invention can determine the development of the tissue in real time.

Furthermore, the observable increase in the speed of sound in structures with cells also indicates that the component K of the speed of sound equation increased more than the increase in density, which implies a higher elastic modulus or lower viscosities (v).

Perhaps the most interesting result is the asymptotic density value of 1300 kg·m$^{-3}$ obtained for cell-laden structures, which could be explained due to a decrease in nutrient support associated with higher shear stresses. But curiously, the value obtained is quite similar to the results in the literature for collagen (1.3 g·cm$^{-3}$), thus suggesting that the final density achieved is composed mainly of collagen.

**Claims**

1. A method for determining in real time the growth and maturation of a biological tissue, comprising:

   - introducing a flow of culture medium into a bioreactor, wherein the bioreactor comprises:

       ◦ a culture chamber (1) that houses a structure (5), which accommodates a cell source,
       ◦ a transmitting transducer (2) and a receiving transducer (3) located opposite the transmitting transducer (2), and
       ◦ a fluid circuit (4) connected to the culture chamber (1), located between the transmitting transducer (2) and the receiving transducer (3), and through which the flow of culture medium destined to pass through the structure (5) is introduced;

   - generating an ultrasonic wave by means of the transmitting transducer (2) that passes through the culture chamber (1) and the structure (5) with the cell source housed in said culture chamber (1);
   - receiving the ultrasonic wave in the receiving transducer (3) once it has passed through the structure (5), generating a reception signal (3);
   - generating an ultrasound propagation simulation model, calculating a simulated reception signal and setting density of the structure (5) and speed of sound in the structure (5) as dependent variables;
   - defining a distance metric between the simulated reception signal and the reception signal (3) of the ultrasonic wave obtained in the receiving transducer (3),
   - determining the density in the structure (5) and the speed of sound in the structure (5) that minimises the distance metric, at each moment of time; and
   - correlating the density and speed of sound that are obtained with an increase in stiffness and tissue content, thus monitoring tissue growth over time.

2. The method for determining in real time the growth and maturation of a biological tissue according to claim 1, wherein the simulation model is generated using finite element models, finite differences, a transfer-matrix method (transfer-matrix formalism) or spectral techniques.

3. The method for determining in real time the growth and maturation of a biological tissue according to any of claims 1 to 2, wherein the distance metric used is a cross-correlation between the signals, a sum of the components of the vector or a square root of the sum of the squares of the components of the vector.

4. The method for determining in real time the growth and maturation of a biological tissue according to any of claims 1 to 3, wherein the step of determining the density and propagation speed is carried out using a search algorithm that may be a gradient-based method, a genetic algorithm or a neural network trained with a set of pre-calculated receiving ultrasound signals and values of density and speed of sound corresponding to said signals.

5. The method for determining in real time the growth and maturation of a biological tissue according to any of claims 1 to 4, wherein the culture medium further comprises cells of the immune system.

6. The method for determining in real time the growth and maturation of a biological tissue according to any of claims 1 to 5, wherein the cell source is selected from stem cells and differentiated cells.

7. The method for determining in real time the growth and maturation of a biological tissue according to any of claims 1 to 6, wherein the cell source is made of stem cells selected from the list consisting of: adult stem cells, embryonic stem cells and induced pluripotent stem cells.

8. The method for determining in real time the growth and maturation of a biological tissue according to any of claims 1 to 7, wherein the cell source is made of differentiated cells selected from the list consisting of fibroblasts, chondrocytes, chondroblasts, osteocytes, osteoblasts, myocardiocytes, endothelial cells and myocytes.

9. The method for determining in real time the growth and maturation of a biological tissue according to any of claims 1 to 8, wherein the biological tissue is selected from the list consisting of cartilage, bone, muscle, liver tissue, vascular tissue and skin.

10. The method for determining in real time the growth and maturation of a biological tissue according to any of claims 1 to 9, wherein the structure (5) is made up of fibres with a diameter between 50 $\mu$m and 400 $\mu$m and is represented in the simulation model as a matrix of circles.

11. The method for determining in real time the growth and maturation of a biological tissue according to any of claims 1 to 10, wherein the transmitting transducer (2) and the receiving transducer of the structure (5) are facing each other and placed perpendicular to the fluid circuit (4).

12. A method for optimising the production of biological tissue comprising the steps of:

- determining a density of the structure (5) and a speed of sound in the structure (5) using the method according to any of claims 1 to 11; and
- determining a pump flow that optimises the growth and maturation of tissue through the expression:

$$q = u \cdot (1 - \frac{\pi \cdot (K_4 \cdot \rho_{ECM})^2}{4 \cdot A_{total}})$$

where q is the pump flow, u is the flow speed and is constant, $K_4$ is a constant that relates a variation in the diameter of fibres of the structure with a matrix density, $\rho_{ECM}$ is the extracellular matrix density and $A_{total}$ is the total area of the structure (5) in a direction perpendicular to the pump flow.

13. The method for optimising the production of tissue according to claim 12, where $K_4$, which relates a variation in the diameter of fibres of the structure with a matrix density, is obtained through the expression:

$$K_4 = \left(K_2 + \frac{K_3}{K_1}\right)$$

where $K_4$, is obtained from the expressions:

$$\frac{dD_f(t)}{dt} = K_2 \cdot \rho_{ECM} + K_3 \cdot [cell] - \zeta_{material}$$

$$\rho_{ECM} = K_1[cell]$$

and where $K_1$ is a previously determined constant that depends on cell metabolism, the type of matrix synthesised and the age of the cells, [cell] is the concentration of cells attached to the structure, $D_f$ is the diameter of the fibre of the structure and varies depending on: $K_2 \cdot \rho_{ECM}$ which represents the matrix synthesised by cells, where $K_2$ is the diameter increase coefficient as a function of the amount of matrix, $K_3 \cdot [cell]$ which represents the number of attached cells, where $K_3$ is the diameter increase coefficient as a function of the cell mass, and $\zeta_{material}$ which represents the degradation of the polymer and is considered negligible.

14. A bioreactor for generating biological tissue, which is intended to accommodate a cell source and comprises:

- a cell culture chamber (1) configured to house a structure (5) to accommodate the cell source;
- a transmitting transducer (2) configured to emit an ultrasonic wave in the direction of the structure (5);
- a receiving transducer (3) arranged opposite the transmitting transducer (2) and configured to receive the ultrasonic wave once it has passed through the structure (5);
- a fluid circuit (4) intended to pass through the structure (5) in order to generate a shear stress on the surface thereof to facilitate the generation and differentiation of cells; wherein the fluid circuit (4) comprises an inlet (6) and an outlet (7), and a peristaltic pump (8) configured to generate a laminar flow; and
- a processing module configured to carry out the steps of the method according to any of claims 1 to 11, and depending on the density in the structure (5) and the speed of sound in the structure (5) that are obtained, controlling the flow generated by the peristaltic pump (8) by regulating the generated pump flow, according to the steps of the method according to any of claims 12 to 13.

15. The bioreactor for generating a biological tissue according to claim 14, which further comprises a signal generator (9) connected to the transmitting transducer (2).

16. The bioreactor for generating a biological tissue according to any of claims 14 or 15, which further comprises an amplifier (10) and an oscilloscope (11) that are connected to the receiving transducer (3).

17. The bioreactor for generating a biological tissue according to any of claims 14 to 16, the processing module is a Raspberry Pi (12) and an Arduino board (13) connected to the peristaltic pump (8).

18. The bioreactor for generating a biological tissue according to any of claims 14 to 17, which further comprises a container (14) intended to store the culture medium and connected to the peristaltic pump (8), to the inlet (6) of the fluid circuit (4) and to the outlet (7) of the fluid circuit (4).

19. The bioreactor for generating a biological tissue according to any of claims 14 to 18, the culture chamber (1) is made of a material selected from the list consisting of: polymethyl methacrylate (PMMA), stainless steel, titanium, sapphire crystal, platinum, and glass.

20. A system for generating a biological tissue that comprises the bioreactor of any of claims 14 to 19, and further comprises a structure (5) located in the cell culture chamber (1), on which the cell source is arranged.

21. The system for generating a biological tissue according to claim 20, wherein the structure (5) is made of a polymer selected from the list consisting of: 1,4-butanediol thermoplastic polyurethane (bTPUe), polylactic acid (PLA), polycaprolactone (PLC) and polylactic-co-glycolic acid (PLGA).

22. The system for generating a biological tissue according to any of claims 20 or 21, wherein the structure (5) is made up of fibres with a diameter of between 50 $\mu$m and 400 $\mu$m.

23. The system for generating a biological tissue according to any of claims 20 to 22, wherein the surface of the structure (5) is functionalised with Arg-Gly-Asp (RGD) peptides, with PBA, 1-pyrene acetic acid (PCA) with fibronectin or with collagen.

24. A computer program for determining in real time the growth and maturation of a biological tissue configured to carry out the steps of the method according to any one of claims 1 to 11 and for optimising the production of tissue configured to carry out the steps of the method according to any one of claims 12 to 13, using the system according to any one of claims 20 to 23.

25. A storage unit configured to store the computer program according to claim 24.

FIG. 1

FIG. 2B

FIG. 2A

**FIG. 2C**

**FIG. 2D**

**FIG. 2E**

FIG. 2F

FIG. 3A

FIG. 3B

FIG. 3C

**FIG. 3D**

**FIG. 3E**

FIG. 4A

FIG. 4B

FIG. 4C

FIG. 4D

**Control**       **BR**

## FIG. 4E

**Control**       **BR**

40 nm/div       100 nm/div

## FIG. 4F

**FIG. 4G**

**FIG. 4H**

**FIG. 5A**

**FIG. 5B**

FIG. 5C

FIG. 5D

**Cells**

FIG. 6A

**Blank**

FIG. 6B

FIG. 6C

FIG. 6D

FIG. 6E

FIG. 6F

FIG. 6G

**FIG. 6H**

**REFERENCES CITED IN THE DESCRIPTION**

**Non-patent literature cited in the description**

- **OGUNRINADE, O.** ; **KAMEYA, G. T.** ; **TRUSKEY, G. A.** Effect of fluid shear stress on the permeability of the arterial endothelium. *Annals of biomedical engineering*, 2002, vol. 30, 430-446
- **YEATTS, A. B.** ; **FISHER, J. P**. Bone tissue engineering bioreactors: dynamic culture and the influence of shear stress. *Bone*, 2011, vol. 48 (2), 171-181

- **LEE, A. A.** ; **GRAHAM, D. A.** ; **DELA CRUZ** ; **S., RATCLIFFE, A.** ; **KARLON, W. J.** Fluid shear stress-induced alignment of cultured vascular smooth muscle cells. *J. Biomech. Eng.*, 2002, vol. 124 (1), 37-43
- **HUANG, Y.** ; **JIA, X.** ; **BAI, K.** ; **GONG, X.** ; **FAN, Y.** Effect of fluid shear stress on cardiomyogenic differentiation of rat bone marrow mesenchymal stem cells. *Archives of medical research*, 2010, vol. 41 (7), 497-505